# EUROPEAN PATENT APPLICATION

(11) **EP 4 082 396 A1**
(43) Date of publication of application: **02.11.2022**
(21) Application number: 20906960.8
(22) Date of filing: 11.12.2020
(51) Int. Cl.: A47C 17/04

(54) **OSCILLATING SLEEP INDUCTION BED**

(30) Priority: 23.12.2019 JP 2019231830
(71) Applicant: Kamei, Masamichi, Tokyo 153-0062 (JP)
(72) Inventor: Kamei, Masamichi, Tokyo 153-0062 (JP)
(74) Representative: TBK
(86) International application number: PCT/JP2020/046281
(87) International publication number: WO 2021/131793

(57) **Abstract**

Provided is an exceptionally safe oscillating sleep induction bed capable of smoother oscillation to address problems such as the stability of an oscillation mechanism and the complexity of a drive mechanism in conventional sleep induction beds. This oscillating sleep induction bed (1) comprises a plurality of oscillating support devices (10) positioned in a dispersed manner so as to support a bed body (1a) in a manner allowing oscillation in the lateral direction of the bed. Each oscillating support device (10) is provided with a support part (11) installed on the floor surface, a pendulum member (31) capable of oscillating in the lateral direction of the bed, and link members (21) that link the upper part of the support part (11) and the lower part of the pendulum member (31). The upper end part of the pendulum member (31) is fixed to the lower surface side of the bed body (1a). The pendulum member (31) oscillates with respect to the support part (11), whereby the bed body (1a) oscillates in the lateral direction. A drive device (40) is provided between the bed body (1a) and the upper part of the support part (11) constituting the oscillating support device (10), whereby the bed body (1a) can be made to oscillate laterally with respect to the support part (11).

## Description

### TECHNICAL FIELD

This invention relates to an oscillating sleep induction bed with which fall-asleep of a user can be promoted.

### BACKGROUND ARTS

Heretofore, a vibratory bed in which micro vibration is applied by a vibrating device embedded in a mattress part of the bed and an oscillatory bed in which the bed itself is subjected to oscillation have been developed.

As one oscillatory bed for sleep promotion with which fall-asleep can be promoted in such a way that comfortable oscillation necessary for a person to fall asleep is applied to the body of the person, while vertical oscillation given to the head thereof is being minimized, a patent document 1 below discloses an oscillatory bed for sleep promotion which comprises a bed base, an oscillating frame supported oscillatably by the bed base, a bed floor supported by the oscillating frame, and an oscillation drive means for driving and oscillating the oscillating frame together with the bed floor, wherein the oscillating frame is constituted such that its oscillatory center axis extending along the height direction of a user in a posture of lying on one's back on the bed floor passes longitudinally through the head of the user.

A patent document 2 below discloses an oscillatory bed which comprises a base, an oscillating body supported in a manner allowing reciprocating motion in the bed width direction, a drive device mounted on the base to reciprocate the oscillating body, and a bed frame for a sleeping person to be placed thereon, wherein comfortable sleep is provided in such a way that comfortable rolling stimulus and/or motion are given to the muscles and/or joints of the sleeping person centering on one's back, basically provided that a back bed body part for the back of the sleeping person to be placed thereon is subjected to reciprocating motion in the bed width direction.

A patent document 3 below discloses a bed with a sleep inducement function in which a vibration board installed on the bed is provided with a continuous micro-vibration generator for generating continuous micro vibration like vibration of a running train, for instance, and an intermittent single-shot vibration generator for generating single-shot vibration repeated at a substantially constant period like vibration generated at rail connections, for instance, wherein satisfactory sleep inducement action is provided in such a way that both the continuous micro-vibration and the intermittent single-shot vibration are allowed to multiply act on the human body lying on the bed.

As one oscillatory bed for giving massaging and/or rehabilitation treatments to a user and/or promoting sleep of the user lying on the bed, a patent document 4 below discloses a human body oscillating device which comprises a fixing section arranged in an installation place, a section to be oscillated arranged thereon, an oscillation drive section for reciprocating the section to be oscillated, a first slide mechanism for causing the section to be oscillated to oscillate relative to the fixing section along the direction of oscillation, a second slide mechanism for causing a vertical plane to regulate horizontal shift of the section to be oscillated, a control section for controlling each drive section, various sensors and an operation panel.

In relation to a rocking mechanism of a rocking chair without regard to an oscillatory bed, a patent document 5 below discloses a rocking mechanism structure which is provided with a narrow box-shaped armrest with a bottom face opened, the armrest being provided at either side of a seat plate of a chair which performs rocking motion and in which a chair body is pivoted and connected to a lower end of a rocking link suspended from an upper end of a support frame uprightly installed on either side of a base of the chair, wherein at least an upper part of the support frame and that of the link are housed in a box part of the armrest.

As one rocking chair which performs smooth rocking motion, a patent document 6 below discloses a rocking chair which comprises a left and right pair of leg members extending in the longitudinal direction, a seat base member arranged above the pair of leg members to place a seat thereon, a left and right pair of side plate members extending downwards from the seat base member, a left and right pair of arm members extending from the respective leg members toward the seat base member, a left and right pair of front link members, each of which links an upper front part of each arm member and a lower front part of each side plate member together in a longitudinally rotatable manner, and a left and right pair of rear link members, each of which links an upper rear part of each arm member and a lower rear part of each side plate member together in a longitudinally rotatable manner, wherein the rocking chair is provided with first electromagnets respectively fixed to the arm members and second electromagnets respectively fixed to the seat base member so as to be opposed to the first electromagnets in the lateral direction.

A patent document 7 below discloses a health bed which is provided with a laterally oscillating board on the upper surface of a bed body, wherein the laterally oscillating board is subjected to reciprocating motion in the lateral direction. This health bed is constituted such that the reciprocating motion of the laterally oscillating board is performed in such a way that rotation of a varying speed motor fixed to the bed body is transmitted to a link rod having the lower end pivotally attached to the bed body, with the upper end pivotally attached to the laterally oscillating board.

A patent document 8 below discloses a relaxation device in which a base part for supporting the seat surface of a rocking chair is supported oscillatably through two link members and crank members, and alternate normal/reverse rotation and one-way rotation of a motor provided to serve as an oscillation drive means are controlled in accordance with the amplitude of oscillation, thereby stabilizing an oscillating speed.

A patent document 9 below discloses a sleep induction bed which comprises a support frame body of the bed, a bed surface member made to slide on the upper surface of the support frame body, and a gravitational vibrator for applying a drive force, wherein this sleep induction bed is constituted such that the support frame body and the bed surface member are supported oscillatably by a plurality of link members.

A patent document 10 below discloses a mechanical-type electric swing bed in which a board part provided to serve as an upper support member of the bed is supported oscillatably on a base provided to serve as a lower support member of the bed, wherein the board part of the bed constitutes a support structure in which this board part is suspended and supported through both a strut uprightly installed on a horizontal member constituting the base of the bed and a suspension member provided on an arm at an upper end of the strut.

A non-patent document 1 below states experimental results showing that, in verification of a relaxation effect related to fall-asleep helping effect and/or sleepy feeling according to a control experiment having been conducted based on the contrast between application of rhythm stimulus of about 0.33 to 0.67Hz (about 30 times per minute) for 10 minutes and no application of rhythm stimulus, it is proved from this control experiment that such application of rhythm stimulus brings about a reduction in awakening consciousness level, so that a physiological and subjective relaxation effect is obtained. This non-patent document also contains references stating that a rhythmical heart rate of a pregnant woman in healthy and emotional stable conditions are said to be comparable to sounds and vibrations capable of giving ease to an unborn baby and also that rhythmical handclapping presents "1/f fluctuation" that is said to give comfortable feeling to a person.

### PRIOR ART DOCUMENTS

### PATENT DOCUMENTS

Patent document 1: Japanese Patent Publication No. 4590238
Patent document 2: Japanese Patent Publication No. 5086696
Patent document 3: Japanese Unexamined Patent Application Publication No. 2004-024678
Patent document 4: Japanese Unexamined Patent Application Publication No. 2018-139766
Patent document 5: Japanese Unexamined Patent Application Publication No. 2001-070075
Patent document 6: Japanese Unexamined Patent Application Publication No. Hei 11-276286
Patent document 7: Microfilm of Japanese Utility Model Application No. Sho 49-013113 (Japanese Unexamined Utility Model Application No. Sho 50-104804)
Patent document 8: International Publication No. 2009/128361
Patent document 9: Specification of Chinese Patent Application Publication No. 105661993
Patent document 10: Japanese Registered Utility Model Publication No. 3122978

### NON-PATENT DOCUMENT

Non-patent document 1: Sumi HIROI et al, 2010, Effects of Periodically Rhythmical Stimulus for Ten Minutes on Awakening Consciousness Level, Japanese Society of Nursing Technology Journal in Japanese Society of Nursing Technology, Vol.9, No. 2, pp 29 to 38

### SUMMARY OF THE INVENTION

### PROBLEMS TO BE SOLVED BY THE INVENTION

The oscillatory bed for sleep promotion as disclosed in the patent document 1 constitutes a structure in which the oscillating frame with the bed floor mounted thereon is laterally oscillated by the oscillation drive means in state where the oscillating frame is axially supported on a support shaft of a side frame placed at the front and rear ends of the bed through a triangular suspension arm. The oscillation drive means is constituted such that a brake motor fixedly supported by an intermediate part of a bottom frame of the bed base is provided to serve as a drive source for driving and oscillating the oscillating frame laterally with a prescribed amplitude through an eccentric plate, an eccentric shaft, a connecting rod and an oscillating arm, for instance.

In the case of the oscillatory bed for sleep promotion as disclosed in the patent document 1, the bed floor for the user to be placed thereon in a lying posture is designed such that the front and rear ends of the bed floor are only axially supported through the triangular suspension arm relatively to the support shaft of the front and rear side frames, so that all load shall be applied to a connection part between the support shaft and the suspension arm of the bed floor, thereby causing an increase in bending stress and/or shearing stress acting on the bed floor or the oscillating frame when the user turns over and moves toward one side of the bed in one's sleep, for instance, thus resulting in the difficulty in meeting the design standard for safety.

Besides, for the oscillation drive means used for the oscillatory bed for sleep promotion as disclosed in the patent document 1, there exist the problems such as the occurrence of a large gap space between the bed base in a lower position and the bed floor in an upper position, in addition to upsizing of the bed as a whole, because of the presence of a large space for installation of the brake motor, the eccentric plate, the eccentric shaft, the connecting rod, the oscillating arm and the oscillating frame.

The oscillatory bed with the sleep inducement function as disclosed in the patent document 3 makes use of the continuous micro-vibration generator for applying the vibration like the vibration of the running train, together with the intermittent single-shot vibration generator for applying the vibration like the vibration generated at the rail connections, in which case, however, it is not clear how low-frequency vibratory motion is applied to which part of the body by the vibration generators, and besides, the resultant effect is not clearly stated.

The oscillatory bed as disclosed in the patent document 4 is constituted such that a structure for longitudinal oscillation of the bed includes the means for regulating the horizontal shift of the section to be oscillated, in which case, however, any oscillating motion other than the longitudinal oscillation is not taken into consideration.

The beds as disclosed in the patent documents 7, 9 and 10 are different in oscillating mechanism, but are basically constituted such that the surface member installed on the upper surface of the bed body supported on the floor surface or that of the support frame is made to oscillate on the upper surface of the bed body or that of the support frame, and as a result, a load in regards to the drive force is enlarged.

The devices as disclosed in the patent documents 5, 6 and 8 are those relating to the rocking chair and cannot be applied intact to the bed for the person to be placed thereon in a lying posture.

To address problems such as the stability of the oscillating mechanism and the complexity of the drive mechanism in the above conventional sleep induction beds, the present invention is aimed at providing an exceptionally safe oscillating sleep induction bed capable of smoother oscillation in such a way as to contrive a structure in which a part of a rocking mechanism of a rocking chair using a link member is incorporated in an oscillatory bed.

### MEANS FOR SOLVING THE PROBLEMS

According to the present invention, an oscillating sleep induction bed comprises a bed body and an oscillating support device for supporting the bed body in a manner allowing oscillation in the lateral direction of the bed, wherein the oscillating support device is provided with a support part installed on the floor surface, a pendulum member capable of oscillating in the lateral direction of the bed and a plurality of spacedly arranged link members in which both ends thereof at which an upper part of the support part and a lower part of the pendulum member are linked are in the form of pin joint structure, and wherein an upper end part of the pendulum part is fixed to the lower surface side of the bed body.

The oscillating support device is capable of increasing the supporting stability of the whole of the bed in such a way as to arrange a plurality of oscillating support devices in a dispersed manner relatively to the bed body. These oscillating support devices may be arranged respectively in positions close to the four corners of the bed, for instance, or alternatively, at the longitudinal front and rear sides of the bed (or in the direction of the body of a user), or otherwise, in an intermediate position, in addition to the front and rear sides.

The pendulum member is preferably installed at the front and rear sides of the support part in the longitudinal direction of the bed in consideration of the stability and the balance, or alternatively, can be installed at either of the front and rear sides from the viewpoint of structure, or otherwise, between a plurality of members constituting the support part in a manner of being sandwiched therebetween.

In the oscillating sleep induction bed of the present invention, it is necessary to constitute a structure in which each support part is structurally stabilized, when this oscillating sleep induction bed is supposed to have a structure in which the bed body and the weight of the user lying thereon are supported by the plurality of support parts arranged in the dispersed manner.

As such support structure, it is possible to adopt structures such as a structure in which each support part is formed in the shape of a rectangular parallelepiped support frame body composed of a pair of lower horizontal materials extending in the direction of oscillation, another pair of lower horizontal materials extending in a direction orthogonal to the direction of oscillation and connecting the pair of lower horizontal materials in the direction of oscillation together, four columnar materials uprightly installed on the lower horizontal materials in the direction of oscillation, a pair of upper horizontal materials extending in the direction of oscillation and installed on the upper surface of the columnar materials, and another pair of upper horizontal materials extending in a direction orthogonal to the direction of oscillation and connecting the pair of upper horizontal materials in the direction of oscillation together.

The oscillating sleep induction bed of the present invention can be subjected to oscillation also by manual operation when no drive device is used, for instance, but is basically constituted such as to be subjected to oscillation by some kind of drive device, wherein the drive device is operated to oscillate the bed, and as a result, the user lying on the bed can be induced to sleep efficiently under the control of the drive device.

The drive means can be of the type in which application of oscillation is performed from the side of the bed body, or alternatively, use can be made of a drive device of the type in which transmission of horizontal vibrational force is performed between the bed body and the upper part of the support part constituting the oscillating support device, for instance, whereby it is possible to eliminate the need for special installation space and also to constitute a compact structure.

Further, the drive device may be installed in a pendulum member position or a link member position to apply horizontal force in the lateral direction of the bed through the pendulum member or the link member.

In the oscillating sleep induction bed of the present invention, a mattress used for a normal bed can be installed on the bed body.

As to the amplitude D (half amplitude) in the reciprocating motion at the time when the bed body is subjected to oscillation by the drive device, it is considered that this amplitude D is limited to at most about 10cm in providing safe and comfortable sleep, and accordingly, it is preferable to perform amplitude adjustment such that the amplitude D can be set to a value about within the range of 1.5cm≦D≦ 8cm.

This is because too small amplitude causes a reduction in oscillating effect, whereas too large amplitude brings about application of a force of acceleration in relation with the vibration frequency and makes it impossible to hold the calm state. Meanwhile, when the amplitude D is about 8cm or 10cm, it is relatively easy to provide designing in relation with the drive mechanism, and besides, contact with the object in the periphery of the bed, for instance, hardly cause any factor of the problem.

As to the vibration frequency f in the reciprocating motion of the bed body, it is considered that this vibration frequency f is limited to at most about 0.8Hz in providing safe and comfortable sleep, while being dependent on the relation with the amplitude, and accordingly, it is preferable to perform vibration frequency adjustment such that the vibration frequency f can be set to a value about within the range of 0.1Hz≦f≦0.7Hz. This is because too small vibration frequency causes a reduction in oscillating effect, whereas too large vibration frequency brings about application of a force of acceleration in relation with the vibration frequency and makes it impossible to hold the calm state.

For instance, the vibration frequency of 0.15Hz is comparable to 9 times of reciprocating motion (9T) for one minute so that the timing (half period of vibration) of shifting to one side will be about as much as a breathing level of the adults at normal times. Likewise, the vibration frequency of 0.6Hz is comparable to 36 times of reciprocating motion for one minute so that the timing (half period of vibration) of shifting to one side amounts to 72 times for one minute and will be about as much as a heart rate level in the calm state. This range of vibration frequency can be considered to be preferable also based on the contents of the above non-patent document 1 such as descriptions on the experimental result showing the reduction in awakening consciousness level caused by the rhythm stimulus of 0.33 to 0.67Hz, on the relaxation effect, on the ease given to the unborn baby by the rhythmical heart rate of the pregnant woman in the healthy and emotional stable conditions, and on the comfortable feeling given by rhythmical handclapping.

The drive device in the case of using the drive means is not specified in type in particular, but can be a drive device equipped with a linear servo motor (inclusive of a linear actuator and a shaft motor, for instance) or with a ball screw and a rotational motor in which rotational motion is applied to the ball screw.

In use of the linear servo motor or the ball screw, the horizontal oscillation of the oscillating section can be realized in the manner of linear reciprocating motion produced by a compact mechanism, and besides, it is easy to perform variable control of the amplitude and/or the vibration frequency. Further, for arrangement of a plurality of linear servo motors or ball screws, it is easy also to synchronize and control these linear servo motors or ball screws.

### EFFECTS OF THE INVENTION

To address the problems such as the stability of the oscillating mechanism and the complexity of the drive mechanism in the conventional sleep induction beds, the oscillating sleep induction bed of the present invention makes use of, as the oscillating support mechanism for supporting the bed body, the oscillating support device provided with the support part installed on the floor surface, the pendulum member capable of oscillating in the lateral direction of the bed, and the plurality of spacedly arranged link members in which both ends thereof at which the upper part of the support part and the lower part of the pendulum member are linked are in the form of pin joint structure, and as a result, it is possible to provide an oscillating sleep induction bed which secures exceptionally smoother oscillating motion and is high in stability as the support mechanism.

The above oscillating support mechanism makes it possible to provide smooth oscillation only by application of a slight force, and as a result, it is possible to perform control of oscillation with a small-output and downsized compact drive device.

### BRIEF DESCRIPTION OF THE DRAWINGS

[FIG. 1] FIG. 1 shows one embodiment of an oscillating sleep induction bed according to the present invention, FIG. 1(a) being a vertical cross-sectional view in the short-length direction, and FIG. 1(b) being a vertical cross-sectional view in the longitudinal direction.
[FIG. 2] FIG. 2 is an exploded perspective view showing an oscillating support mechanism in the embodiment shown in FIG. 1.
[FIG. 3] FIG. 3 is a front view in the short-length direction of the oscillating support mechanism in the embodiment shown in FIG. 1.
[FIG. 4] FIG. 4 is a front view in the longitudinal direction of the oscillating support mechanism in the embodiment shown in FIG. 1.

### MODE FOR EMBODYING THE INVENTION

Hereinafter will be described the present invention with reference to the attached drawings.

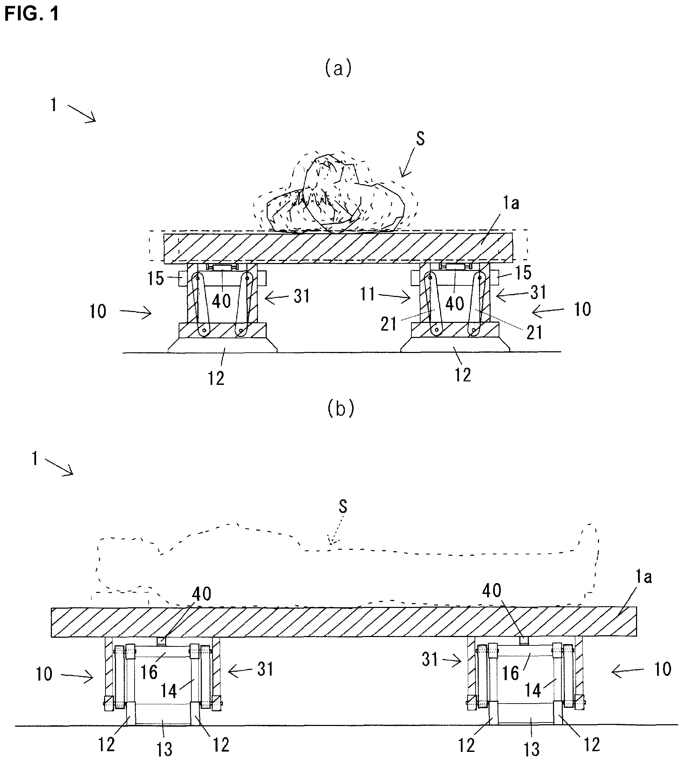

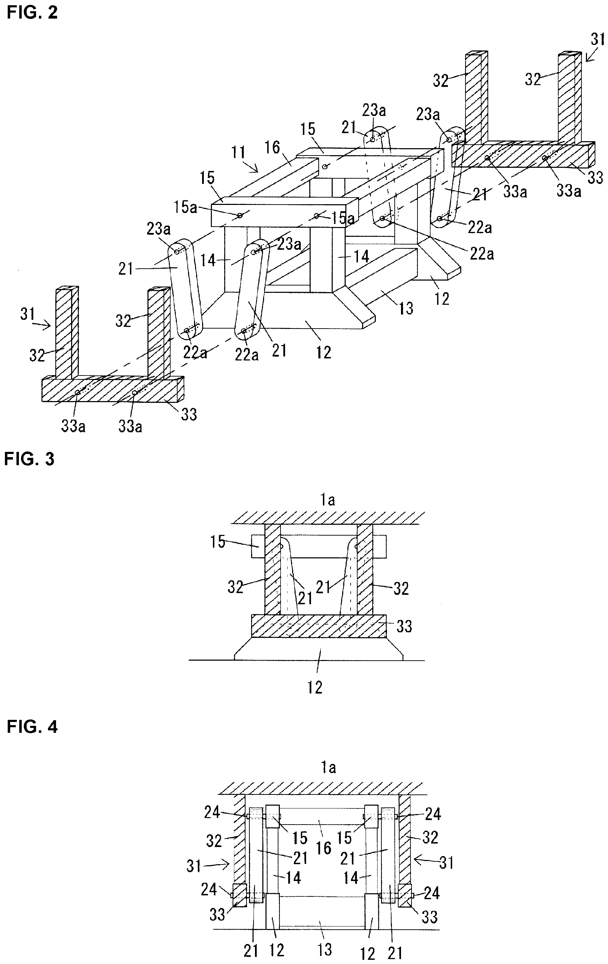

FIGS. 1 to 4 respectively show one embodiment of an oscillating sleep induction bed of the present invention, FIG.1(a) being a vertical cross-sectional view in the short-length direction, FIG. 1(b) being a vertical cross-sectional view in the longitudinal direction, FIG. 2 being an exploded perspective view showing an oscillating support mechanism, FIG. 3 being a front view in the short-length direction of the oscillating support mechanism, and FIG. 4 being a front view in the longitudinal direction of the oscillating support mechanism.

An oscillating sleep induction bed 1 of the present invention has a bed body 1a and an oscillating support device 10 for supporting the bed body 1a in a manner allowing oscillation in the lateral direction of the bed. In this embodiment, the oscillating support device 10 is provided in four corner positions of the bed body 1a in total, so that four oscillating devices 10 are supposed to support the bed body 1a stably, while making it possible to oscillate the bed body 1a.

The oscillating support device 10 is provided with a support part 11 installed on the floor surface, a pendulum member 31 capable of oscillating in the lateral direction of the bed and a link member 21 that links an upper part of the support part 11 and a lower part of the pendulum member 31. In this embodiment, the link member 21 and the pendulum member 31 are installed at the front and rear sides of the support part 11 to increase the stability and also to keep the balance.

As to the link member 21 that links the upper part of the support part 11 and the lower part of the pendulum member 31, there are provided a plurality of spacedly arranged link members, more specifically, two link members respectively at the front and rear sides of the support part according to this embodiment, and both ends of each link member 21 are in the form of pin joint structure.

In this embodiment, the support part 11 is formed in the shape of a rectangular parallelepiped support frame body composed of a pair of lower horizontal materials 12 extending in the direction of oscillation, another pair of lower horizontal materials 13 extending in a direction orthogonal to the direction of oscillation and connecting the lower horizontal materials 12 in the direction of oscillation together, four columnar materials 14 uprightly installed on the lower horizontal materials 12 in the direction of oscillation, a pair of upper horizontal materials 15 extending in the direction of oscillation and installed on the upper surface of the columnar materials 14 and another pair of upper horizontal materials 16 extending in a direction orthogonal to the direction of oscillation and connecting the pair upper horizonal materials 15 in the direction of oscillation together.

With the pendulum member 31 subjected to pin joint to the upper part of the support part 11 constituting the rectangular parallelepiped support frame body, the upper end part of the pendulum member 31 is fixed to the lower surface side of the bed body 1a. By so doing, the pendulum member 31 oscillates with respect to the support part 11, whereby the bed body 1a oscillates in the lateral direction.

In this embodiment, as shown in FIG. 1., a drive device 40 is provided between the bed body 1a and the upper part of the support part 11 constituting the oscillating support device 10, whereby the bed body 1a supported oscillatably through the link member 21 and the pendulum member 31 is made to oscillate laterally with respect to the support part 11. As the drive device 40, use can be made of a linear servo motor, for instance.

As to the amplitude D (half amplitude) in the reciprocating motion at the time when the bed body 1a is subjected to oscillation by the drive device 40, amplitude adjustment is performed such that this amplitude D can be set to at most about 10cm, preferably, a value about within the range of 1.5cm**≦**D**≦**8cm. Likewise, as to the vibration frequency f in the reciprocating motion of the bed body, vibration frequency adjustment is performed such that this vibration frequency f can be set to at most about 0.8Hz, preferably, a value about within the range of 0.1Hz**≦**f**≦**0.7Hz.

The drive device 40 is operated to oscillate the bed body 40, and as a result, the user S lying on the bed can be efficiently induced to sleep under the control of the drive device.

### EXPLANATION OF REFERENCE NUMERALS

S ... User,
1 ... Oscillatory bed, 1a ... Bed body,
10 ... Oscillating support device,
11 ... Support part, 12 ... Lower horizontal material in direction of oscillation, 13 ... Lower horizontal material in direction orthogonal to direction of oscillation, 14 ... Columnar material, 15 ... Upper horizontal material in direction of oscillation, 15a ... Pin hole, 16 ... Upper horizontal material in direction orthogonal to direction of oscillation,
21 ... Link member, 22 ... Lower hinge, 22a ... Pin hole, 23 ... Upper hinge, 23a ... Pin hole, 24 ... Pin,
31 ... Pendulum member, 32 ... Columnar material, 33 ... Lower horizontal material in direction of oscillation, 33a ... Pin hole, 40 ... Drive device

## Claims

1. An oscillating sleep induction bed comprising a bed body and an oscillating support device for supporting said bed body in a manner allowing oscillation in the lateral direction of the bed, wherein said oscillating support device is provided with a support part installed on the floor surface, a pendulum member capable of oscillating in the lateral direction of the bed, and a plurality of spacedly arranged link members in which both ends thereof at which an upper part of said support part and a lower part of said pendulum member are linked are in the form of pin joint structure, and wherein an upper end part of said pendulum member is fixed to the lower surface side of said bed body.

2. The oscillating sleep induction bed according to claim 1, wherein as to said oscillating support device, a plurality of oscillating support devices is arranged in a dispersed matter with respect to said bed body.

3. The oscillating sleep induction bed according to claim 1, wherein said pendulum member is mounted at the front and rear sides of said support part in the longitudinal direction of the bed.

4. The oscillating sleep induction bed according to claim 1, wherein said support part is formed in the shape of a rectangular parallelepiped support frame body composed of a pair of lower horizontal materials extending in the direction of oscillation, another pair of lower horizontal materials extending in a direction orthogonal to the direction of oscillation and connecting said pair of lower horizontal materials in the direction of oscillation together, four columnar materials uprightly installed on said pair of lower horizontal materials in the direction of oscillation, a pair of upper horizontal materials extending in the direction of oscillation and installed on the upper surface of said columnar materials and another pair of upper horizontal materials extending in a direction orthogonal to the direction of oscillation and connecting said pair of upper horizontal materials in the direction of oscillation together.

5. The oscillating sleep induction bed according to claim 1 or 2, wherein a drive means is provided to apply a horizontal force in the lateral direction of the bed with respect to said bed body.

6. The oscillating sleep induction bed according to claim 1 or 2, wherein a drive means is provided to apply a horizontal force in the lateral direction of the bed with respect to said pendulum member or said link member.
